# EUROPEAN PATENT APPLICATION

(11) **EP 4 014 873 A1**
(43) Date of publication of application: **22.06.2022**
(21) Application number: 20214983.7
(22) Date of filing: 17.12.2020
(51) Int. Cl.: A61B 6/00

(54) **X-RAY IMAGING SYSTEM**

(71) Applicant: Koninklijke Philips N.V., 5656 AG Eindhoven (NL)
(72) Inventor: LEUSSLER, Christoph Günther, 5656 AE Eindhoven (NL); SCHÄFER, Dirk, 5656 AE Eindhoven (NL); FINDEKLEE, Christian, 5656 AE Eindhoven (NL)
(74) Representative: Philips Intellectual Property & Standards

(57) **Abstract**

The present invention relates to an X-ray imaging system (10), comprising:
- an X-ray scanner (20) comprising a rotatable structure, wherein the rotatable structure comprises an X-ray source and an X-ray detector, wherein the X-ray scanner is configured to rotate the rotatable structure to position the X-ray source and the X-ray detector at a plurality of different intentional rotational positions, and wherein the X-ray source is configured to emit X-rays and the X-ray detector is configured to acquire a plurality of X-ray data of an object within a plurality of acquisition periods for the plurality of different determined rotational positions, wherein each X-ray data is acquired within a different acquisition period;
- at least one 3D sensor (30); and
- a processing unit (40);
wherein, the at least one 3D sensor is configured to acquire a plurality of sensor data of the rotatable structure, wherein each sensor data is associated with a different acquisition period;
wherein, for each acquisition period of the plurality of acquisition periods the processing unit is configured to determine position data and/or movement data of the X-ray source and position data and/or movement data of the X-ray detector, wherein the determination comprises utilization of the sensor data of the rotatable structure associated with each acquisition period; and
wherein, the processing unit is configured to determine image data of the object, the determination comprising utilization of: the X-ray data of the object for each acquisition period, the position data and/or the movement data of the X-ray source for each acquisition period, and the position data and/or the movement data of the X-ray detector for each acquisition period.

## Description

### FIELD OF THE INVENTION

The present invention relates to an X-ray imaging system, an X-ray imaging method, as well as to a computer program element and a computer readable medium.

### BACKGROUND OF THE INVENTION

In order to facilitate cone-beam computer tomography (CT) using a rotating X-ray scanner such as a fixed or mobile X-ray C-arm scanner, accurate geometric calibration must be carried out. Such calibration requires a precise determination of X-ray focus and detector positions. During standard geometry calibration procedures, a phantom is scanned with known 3D positions of metal beads. The focus and detector positions can be precisely estimated from the 2D projections of the bead phantom. The calibrated geometry is then used for a patient scan assuming that it is reproducible.

However, due to the weight of a rotational system, such as a C-arm system, mechanical resonances or vibrations appear. Thus, when the C - arm is rotating or during start/stop sequences such vibrations are generated, and aperiodic oscillations can also appear, and these vibrations can violate the assumption of reproducibility required for a patient scan. A current solution against vibrations is the provision of a driving system, that manually or semi-automatically drives the X-ray scanner such as the C-arm system, with a damping mechanism or mechanisms to absorb vibrations, but this does not sufficiently address the effect of the vibrations.

### SUMMARY OF THE INVENTION

It would be advantageous to have improved means of carrying out an X-ray scan of a patient with a rotational X-ray scanner. The object of the present invention is solved with the subject matter of the independent claims, wherein further embodiments are incorporated in the dependent claims. It should be noted that the following described aspects and examples of the invention apply also to the X-ray imaging system, the X-ray imaging method, as well as to a computer program element and a computer readable medium.

In a first aspect, there is provided an X-ray imaging system, comprising:
- an X-ray scanner comprising a rotatable structure, wherein the rotatable structure comprises an X-ray source and an X-ray detector, wherein the X-ray scanner is configured to rotate the rotatable structure to position the X-ray source and the X-ray detector at a plurality of different intentional rotational positions, and wherein the X-ray source is configured to emit X-rays and the X-ray detector is configured to acquire a plurality of X-ray data of an object within a plurality of acquisition periods for the plurality of different determined rotational positions, wherein each X-ray data is acquired within a different acquisition period;
- at least one 3D sensor; and
- a processing unit.

The at least one 3D sensor is configured to acquire a plurality of sensor data of the rotatable structure. Each sensor data is associated with a different acquisition period. For each acquisition period of the plurality of acquisition periods the processing unit is configured to determine position data and/or movement data of the X-ray source and position data and/or movement data of the X-ray detector for the plurality of acquisition periods. The determination comprises utilization of the sensor data of the rotatable structure associated with each acquisition period. The processing unit is configured to determine image data of the object, the determination comprising utilization of: the X-ray data of the object for each acquisition period, the position data and/or the movement data of the X-ray source for each acquisition period, and the position data and/or the movement data of the X-ray detector for each acquisition period.

Thus, in this way the effect of vibrations in a rotating X-ray system such as a C-arm system, that move the source and detector in an unintentional way, and that compromise the generation of volume imagery, can be corrected for.

In an example, the determination of the image data of the object comprises utilization of the X-ray data of the object for each acquisition period to generate a reconstructed image of the object and utilization of the position data and/or the movement data of the X-ray source for each acquisition period and the position data and/or the movement data of the X-ray detector for each acquisition period to correct the reconstructed image of the object to generate a corrected reconstructed image of the object.

In an example, the determination of the image data of the object comprises utilization of the position data and/or the movement data of the X-ray source for each acquisition period and the position data and/or the movement data of the X-ray detector for each acquisition period to generate correction information and utilization of the X-ray data of the object for each acquisition period and the correction information to generate a corrected reconstructed image of the object.

In an example, the determination of the image data of the object comprises simultaneous utilization of the X-ray data of the object for each acquisition period and the position data and/or the movement data of the X-ray source for each acquisition period and the position data and/or the movement data of the X-ray detector for each acquisition period to generate a corrected reconstructed image of the object.

Here, a corrected reconstructed image of the object is a reconstructed image of the object that has been corrected for unintentional vibrational positions/movements of the X-ray source and/or X-ray detector.

In an example, the system comprises a memory storing a model configured with position data or position information and/or with movement data or movement information and/or with X-ray data. The processing unit is configured to implement the model, and the determination of the image data of the object comprises utilization of the model.

In an example, the model is configured to determine geometric and/or kinematic information related to the X-ray scanner, the determination comprising utilization of the position data and/or the movement data of the X-ray source for each acquisition period and the position data and/or the movement data of the X-ray detector for each acquisition period. The geometric and/or kinematic information related to the X-ray scanner takes into account a corrected position and/or a corrected movement of the X-ray source and a corrected position and/or a corrected movement of the X-ray detector for each acquisition period.

In an example, the processing unit is configured to implement an image reconstructor to determine the image data of the object on the basis of the X-ray data of the object for each acquisition period, and the geometric and/or kinematic information related to the X-ray scanner.

In an example, the processing unit is configured to implement an image reconstructor to determine initial image data of the object on the basis of the X-ray data of the object for each acquisition period. The processing unit is configured to determine the image data of the object on the basis of the initial image data of the object, and the geometric and/or kinematic information related to the X-ray scanner.

In an example, the model is configured to determine a corrected position and/or a corrected movement of the X-ray source and a corrected position and/or a corrected movement of the X-ray detector for each acquisition period. The determination comprises utilization of the position data and/or the movement data of the X-ray source for each acquisition period and the position data and/or the movement data of the X-ray detector for each acquisition period.

In an example, the processing unit is configured to implement an image reconstructor to determine the image data of the object on the basis of the X-ray data of the object for each acquisition period, the corrected position and/or the corrected movement of the X-ray source and the corrected position and/or the corrected movement of the X-ray detector for each acquisition period.

In an example, the processing unit is configured to implement an image reconstructor to determine initial image data of the object on the basis of the X-ray data of the object for each acquisition period. The processing unit is configured to determine the image data of the object on the basis of the initial image data of the object, the corrected position and/or the corrected movement of the X-ray source and the corrected position and/or the corrected movement of the X-ray detector for each acquisition period.

In other words, the processing unit determines corrected geometry information that is utilized to generate image data that is corrected for vibrations of the source and/or detector. The processing unit first calculates the corrected geometry information and uses this instead of the intended/expected position/movements of the X-ray detector and source within a normal image reconstructor to generate the image data. Or the processing unit can first generate image data, that has not yet accounted for the source and detector that were not at their intended/expected positions or moving in their intended/expected movements - due to vibrations - and then use the corrected position/movement data of the source and/or detector to calculate image data that has been corrected for such vibrational movement.

In an example, the model is a trained machine learning algorithm.

In an example, the machine learning algorithm was trained on the basis of: a plurality of position data and/or a plurality of movement data of the X-ray source and X-ray detector each associated with an acquisition period and determined from sensor data of the rotatable structure.

In an example, the machine learning algorithm was trained on the basis of: a plurality of position data and/or a plurality of movement data of an X-ray source and X-ray detector of the calibration X-ray scanner each associated with an acquisition period and determined from sensor data acquired by at least one 3D sensor of a rotatable structure of the X-ray scanner that comprises the X-ray source and the X-ray detector acquired by at least one 3D sensor.

Thus, a machine learning algorithm such as a neural network is provided with sensor data from which the positions and/or movements of the source and detector (in other words how they are vibrating) can be determined. The machine learning algorithm in effect generates a vibration calibration that can then be provided with real position and movement data of a source/detector and the correct for a reconstructed image.

In an example, the machine learning algorithm was trained on the basis of: a plurality of X-ray data of a calibration object acquired with the X-ray scanner over a plurality of acquisition periods, a plurality of position data and/or a plurality of movement data of the X-ray source and X-ray detector each associated with an acquisition period and determined from sensor data of the rotatable structure.

In an example, the machine learning algorithm was trained on the basis of: a plurality of X-ray data of a calibration object acquired with a calibration X-ray scanner over a plurality of acquisition periods, a plurality of position data and/or a plurality of movement data of an X-ray source and X-ray detector of the calibration X-ray scanner each associated with an acquisition period and determined from sensor data of a rotatable structure of the X-ray scanner that comprises the X-ray source and the X-ray detector acquired by at least one 3D sensor.

Thus, a machine learning algorithm such as a neural network is provided with X-ray data of a known object and at the same time with sensor data from which the positions and/or movements of the source and detector (in other words how they are vibrating) can be determined, and this information is used to enable the machine learning algorithm to provide as output a correct volume image that matches the known object. This training can be carried out for all the types of operating procedure that the X-ray scanner would undertake, such as starts, stops, speeds and direction of movements. Then when the trained neural network is provided with X-ray data of an unknown object, along with the position and/or movement data derived from sensor data, a reconstruction of the object can be undertaken that accounts for movements of the source and detector.

In an example, the at least one 3D sensor comprises a LIDAR sensor and/or a RADAR sensor.
In a second aspect, there is provided an X-ray imaging method, comprising:
a) positioning an object between an X-ray source and an X-ray detector of a rotatable structure of an X-ray scanner;
b) rotationally moving the X-ray source and the X-ray detector to different intended rotational positions;
c) emitting by the X-ray source X-rays and acquiring by the X-ray detector a plurality of X-ray data of the object over a plurality of acquisition periods and providing the plurality of X-ray data of the object to the processing unit;
d) acquiring by at least one 3D sensor a plurality of sensor data of the rotatable structure, wherein each sensor data is associated with an acquisition period and providing the plurality of sensor data of the rotatable structure to the processing unit;
e) providing the plurality of X-ray data of the object and the plurality of sensor data of the rotatable structure to the processing unit;
f) determining by a processing unit a plurality of position data and/or a plurality of movement data of the X-ray source, wherein each position data and/or each movement data is associated with an acquisition period and the determining comprises utilizing the sensor data of the rotatable structure for that acquisition period;
g) determining by the processing unit a plurality of position data and/or a plurality of movement data of the X-ray detector, wherein each position data and/or each movement data is associated with an acquisition period and the determining comprises utilizing the sensor data of the rotatable structure for that acquisition period; and
h) determining by the processing unit image data of the object, the determining comprising utilizing: the plurality of X-ray data of the object, the plurality of position data and/or the plurality of movement data of the X-ray source, and the plurality of position data and/or the plurality of movement data of the X-ray detector.

According to another aspect, there is provided a computer program element controlling one or more of the systems as previously described which, if the computer program element is executed by a processing unit, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of an X-ray imaging system;
Fig. 2 shows an X-ray imaging method;
Fig. 3 shows a detailed high level system architecture and workflow of an exemplar X-ray imaging system;
Fig. 4 shows a schematic set up of an example of an X-ray imaging system;
Fig. 5 shows a schematic set up of an example of an X-ray imaging system.

### DETAILED DESCRIPTION OF EMBODIMENTS

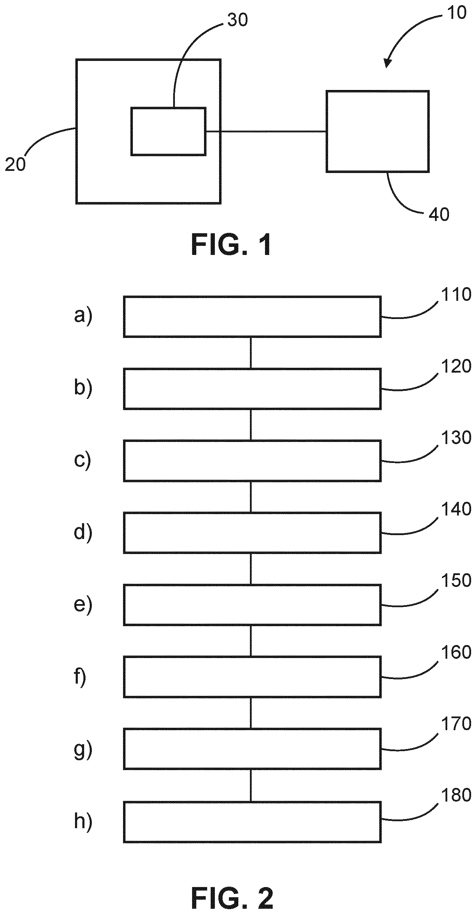

Fig. 1 shows a schematic example of an X-ray imaging system 10. The system 10 comprises an X-ray scanner 20 comprising a rotatable structure. The rotatable structure comprises an X-ray source and an X-ray detector. The X-ray scanner is configured to rotate the rotatable structure to position the X-ray source and the X-ray detector at a plurality of different intentional rotational positions. The X-ray source is configured to emit X-rays and the X-ray detector is configured to acquire a plurality of X-ray data of an object within a plurality of acquisition periods for the plurality of different determined rotational positions. Each X-ray data is acquired within a different acquisition period. The system 10 also comprises at least one 3D sensor 30, and a processing unit 40. The at least one 3D sensor is configured to acquire a plurality of sensor data of the rotatable structure. Each sensor data is associated with a different acquisition period. For each acquisition period of the plurality of acquisition periods the processing unit is configured to determine position data and/or movement data of the X-ray source and position data and/or movement data of the X-ray detector. The determination comprises utilization of the sensor data of the rotatable structure associated with each acquisition period. The processing unit is configured to determine image data of the object, the determination comprising utilization of: the X-ray data of the object for each acquisition period, the position data and/or the movement data of the X-ray source for each acquisition period, and the position data and/or the movement data of the X-ray detector for each acquisition period.

Reference to an acquisition period should be understood also in the sense that there is a time-stamp or other temporal information, enabling sensor data and X-ray data to be able to be correlated/synchronized.

It is also to be noted that the X-ray scanner as well as intentionally rotating the X-ray source and X-ray detector to different determined rotational positions, can if necessary also intentionally move the X-ray detector and/or X-ray source intentionally radially inwards or outwards to determined positions. Therefore, an intentional rotational movement to a determined position could be circular or elliptical of indeed a complex shape. However, the sensor data enables deviations away from an intended/expected movement of the X-ray source and X-ray detector to detected and taken into account when an image is constructed from the acquired X-ray data.

It is to be noted that sensor data associated with an acquisition period can be before, after or during the acquisition period. Also, sensor data associated with an acquisition period can be sensor data captured by more than one sensor at the same time.

In an example, the system comprises a temperature sensor and/or humidity sensor configured to acquired temperature data and/or humidity data that can be provided to the processing unit. The determination of the image data of the object can comprise utilization of the temperature data and/or humidity data.

Thus, the system can take temperature and humidity into account, which can lead to variations in vibrations modes, when constructing the image.

In an example, the X-ray scanner is configured to intentionally rotate the rotatable structure to move the X-ray source and the X-ray detector to different intended rotational positions about an iso-center of the X-ray scanner.

In an example, the processing unit is configured to determine the plurality of position data and/or the plurality of movement data of the X-ray source with respect to the iso-center of the X-ray scanner or another determined position.

In an example, the processing unit is configured to determine the plurality of position data and/or the plurality of movement data of the X-ray detector with respect to the iso-center of the X-ray scanner or another determined position.

In an example, the processing unit is configured to determine the plurality of position data with respect to a plurality of expected positions.

Thus, as the source and detector are rotated, at an acquisition period when X-ray data are acquired, the intended/expected positions of the source and detector are known, but due to vibrations etc, the source and/or detector can be deviated away from this situation. The sensor data is processed to determine this positional/movement deviation.

In an example, the processing unit is configured to determine the plurality of movement data with respect to a plurality of expected movements.

In other words, the position data can be absolute position data in a coordinate system of a room that in effect is aligned with geometric data/information of the X-ray scanner, albeit with slight deviations due to the scanner vibration.

However, geometric data/information can also be utilized, and in which case the position data can be deviations from geometric data / information of the scanner.

Thus, as the source and detector are rotated, at an acquisition period when X-ray data are acquired, the intended/expected movement of the source and detector are known, but due to vibrations etc, the source and/or detector can be deviated away from this situation. For example, a vibrational movement outward or inward can be detected, when none is expected, and a vibrational movement backwards or forwards with respect to the rotational direction can be detected leading to decreased/increased movement speeds. The sensor data is processed to determine this movement deviation.

According to an example, the determination of the image data of the object comprises utilization of the X-ray data of the object for each acquisition period to generate a reconstructed image of the object and utilization of the position data and/or the movement data of the X-ray source for each acquisition period and the position data and/or the movement data of the X-ray detector for each acquisition period to correct the reconstructed image of the object to generate a corrected reconstructed image of the object.

According to an example, the determination of the image data of the object comprises utilization of the position data and/or the movement data of the X-ray source for each acquisition period and the position data and/or the movement data of the X-ray detector for each acquisition period to generate correction information and utilization of the X-ray data of the object for each acquisition period and the correction information to generate a corrected reconstructed image of the object.

According to an example, the determination of the image data of the object comprises simultaneous utilization of the X-ray data of the object for each acquisition period and the position data and/or the movement data of the X-ray source for each acquisition period and the position data and/or the movement data of the X-ray detector for each acquisition period to generate a corrected reconstructed image of the object.

In an example, determination of the image data comprises utilization of the plurality of position data and/or the movement data of the X-ray source and the plurality of position data and/or the movement data of the X-ray detector to back project the plurality of X-ray data of the object during reconstruction of an image of the object.

In an example, the sensor data of the rotatable structure comprises sensor data of the X-ray detector.

In an example, the sensor data of the rotatable structure comprises sensor data of the X-ray source.

Thus, sensor data such as lidar or radar or even high resolution camera imagery can capture data of the X-ray source and X-ray detector directly, from which the positions and orientations of the X-ray source and detector can be determined for each acquisition period. These positons can be absolute positions within a coordinate system of the room within which the X-ray scanner is located, or can be positions with respect to where the X-ray source and X-ray detector would be expected to be, if there was no system vibration. Sensor data from more than one sensor can be used together to determine the position and/or movement data.

However, the sensor data does not need to include the X-ray detector itself and/or the X-ray source itself. The sensor data can be of the parts of rotatable structure away from the source and detector. Thus, sensor data of a fixed location in space of for example a C-arm structure can be acquired, and where the C-arm structure is rotating within this location, with the source moving toward this location and detector moving away from this region. However, the part of the C-arm can be moving or vibrating, and a prior calibration can be utilized to determine or infer movement of the X-ray source and/or X-ray detector. The calibration can be for an X-ray scanner of the same type, and where sensor data of the C-arm structure is acquired and at the same time positon and/or movement data or information of the source and detector acquired. Then, for different movements of the C-arm the sensor data of the C-arm structure away from the detector and source can be correlated to the positions / movements of the source / detector enabling, later sensor data of only the C-arm structure away from the source and detector to be used to determine position / movements of the source and detector.

In an example, determined position data of the X-ray source comprises radial position data, and wherein determined position data of the X-ray detector comprises radial position data.

In an example, determined position data of the X-ray source is with respect to an iso-center of the X-ray scanner, and determined position data of the X-ray detector is with respect to the iso-center of the X-ray scanner.

In an example, determined movement data of the X-ray source is respect to the iso-center of the X-ray scanner, and determined movement data of the X-ray detector is with respect to the iso-center of the X-ray scanner.

In other words, as the X-ray source and X-ray detector are intentionally rotated, calibration information provided by the X-ray scanner can provide the calibrated position of the X-ray source and X-ray detector. However, as the X-ray source and X-ray detector are moved, which can involve accelerations, stops, decelerations, forward-backward motions, in the real world they move/vibrate away from such calibrated positions, and this movement can have inward and outward components, and the sensor data enables such movement/vibrations to be detected and taken into account when constructing an image from the acquired X-ray data.

In an example, determined position data of the X-ray source comprises rotational position data different to the intentional rotational positions of the X-ray source, and wherein position data of the X-ray detector comprises rotational position data different to the intentional rotational positions of the X-ray detector.

In other words, as the X-ray source and X-ray detector are intentionally rotated to different positions, calibration information provided by the X-ray scanner can provide for an intended/expected position of the X-ray source and X-ray detector. However, as the X-ray source and X-ray detector are moved, which can involve accelerations, stops, decelerations, forward-backward motions, in the real world they move/vibrate away from such intended/expected positions, and this movement can have components in the direction of travel or opposite to the direction of travel. In other words, the X-ray detector and X-ray source can be vibrating forwards and backwards as they rotate and even when they have been stopped, where these vibrations are not intended. However, the sensor data enables such movement/vibrations to be detected and taken into account when constructing an image from the acquired X-ray data.

In an example, the X-ray scanner is configured to intentionally rotationally move the X-ray source to different intended radial positions with respect to the iso-center of the X-ray scanner.

In an example, the X-ray scanner is configured to intentionally rotationally move the X-ray detector to different intended radial positions with respect to the iso-center of the X-ray scanner.

In an example, determined position data of the X-ray detector comprises at least one angle between a normal perpendicular to a center of a surface of the X-ray detector and a projected line from the center of the surface of the X-ray detector that goes through the iso-center.

Thus, as the X-ray detector is intentionally rotated, calibration information provided by the X-ray scanner can provide for an intended/expected direction in which the central detection axis of the detector is pointing. However, as X-ray detector is moved, the accelerations, stops, decelerations, forward-backward motions, can lead to the detector twisting or tilting away from this intended/expected situation. This tilting can have components in both detector plane axes and can be vibrational. However, the sensor data enables such tilting motion to be detected and taken into account when constructing an image from the acquired X-ray data.

In an example, determined movement data of the X-ray source comprises radial movement data, and wherein determined movement data of the X-ray detector comprises radial movement data.

In an example, movement data of the X-ray source comprises rotational movement data different to the intended rotational movements of the X-ray source, and wherein movement data of the X-ray detector comprises rotational movement data different to the intended rotational movements of the X-ray detector.

In other words, the processing unit is configured to determine image data taking into account these differences of positioning/movement.

In this manner, the source and detector and intentionally rotated, and can be stopped, started and moved at different speeds, and in doing this the source and detector can move or vibrate with respect to perfect positions, and this vibration movement can be inwards, outwards, and sideways and can be a twisting or tilting of the detector. This, without correction leads to a compromised generated volume image, but by detecting this movement by 3D sensors the effect of this movement can be corrected for.

According to an example, the system comprises a memory storing a model configured with position data or position information and/or with movement data or movement information and/or with X-ray data. The processing unit is configured to implement the model, and the determination of the image data of the object can comprise utilization of the model.

According to an example, the model is configured to determine geometric and/or kinematic information related to the X-ray scanner, the determination comprising utilization of the position data and/or the movement data of the X-ray source for each acquisition period and the position data and/or the movement data of the X-ray detector for each acquisition period. The geometric and/or kinematic information related to the X-ray scanner takes into account a corrected position and/or a corrected movement of the X-ray source and a corrected position and/or a corrected movement of the X-ray detector for each acquisition period.

According to an example, the processing unit is configured to implement an image reconstructor to determine the image data of the object on the basis of the X-ray data of the object for each acquisition period, and the geometric and/or kinematic information related to the X-ray scanner.

According to an example, the processing unit is configured to implement an image reconstructor to determine initial image data of the object on the basis of the X-ray data of the object for each acquisition period. The processing unit is configured to determine the image data of the object on the basis of the initial image data of the object, and the geometric and/or kinematic information related to the X-ray scanner.

According to an example, the model is configured to determine a corrected position and/or a corrected movement of the X-ray source and a corrected position and/or a corrected movement of the X-ray detector for each acquisition period, the determination comprising utilization of the position data and/or the movement data of the X-ray source for each acquisition period and the position data and/or the movement data of the X-ray detector for each acquisition period.

According to an example, the processing unit is configured to implement an image reconstructor to determine the image data of the object on the basis of the X-ray data of the object for each acquisition period, the corrected position and/or the corrected movement of the X-ray source and the corrected position and/or the corrected movement of the X-ray detector for each acquisition period.

According to an example, the processing unit is configured to implement an image reconstructor to determine initial image data of the object on the basis of the X-ray data of the object for each acquisition period. The processing unit is configured to determine the image data of the object on the basis of the initial image data of the object, the corrected position and/or the corrected movement of the X-ray source and the corrected position and/or the corrected movement of the X-ray detector for each acquisition period.

In an example, the processing unit utilizes the position and/or movement data and integrates or maps it in time to generate geometry and/or kinematic information relating to the X-ray scanner. This can provide information on how the scanner is flexing or vibrating in to provide geometric and/or kinematic information that is different to kinematic and/or kinematic information for a scanner with a structure that did not flex or vibrate as it rotated. This information can be utilized to correct a reconstructed image that then accounts for the flexing and vibrations of the scanner structure, providing a generated image that is what would have been acquired for a scanner that had not flexed or vibrated as it rotated and acquired X-ray data of an object that was used to reconstruct the image of the object.

In an example, a model built up from geometric and/or kinematic data of a scanner structure as it rotated under different conditions can be utilized in correcting for the reconstructed image.

In an example, such a model can also be built up on the basis of X-ray data of an ideal known object, thereby providing one way in which the geometric and/or kinematic data can be correlated to image degradation, and thus enabling later acquired geometric and/or kinematic data or information of an unknown object to be utilized in correcting for unintentional flexing and/or vibrations of the scanner structure in a final image.

According to an example, the model is a trained machine learning algorithm.

According to an example, the machine learning algorithm was trained on the basis of: a plurality of position data and/or a plurality of movement data of the X-ray source and X-ray detector each associated with an acquisition period and determined from sensor data of the rotatable structure.

According to an example, the machine learning algorithm was trained on the basis of: a plurality of position data and/or a plurality of movement data of an X-ray source and X-ray detector of the calibration X-ray scanner each associated with an acquisition period and determined from sensor data acquired by at least one 3D sensor of a rotatable structure of the X-ray scanner that comprises the X-ray source and the X-ray detector acquired by at least one 3D sensor.

According to an example, the machine learning algorithm was trained on the basis of: a plurality of X-ray data of a calibration object acquired with the X-ray scanner over a plurality of acquisition periods, a plurality of position data and/or a plurality of movement data of the X-ray source and X-ray detector each associated with an acquisition period and determined from sensor data of the rotatable structure.

According to an example, the machine learning algorithm was trained on the basis of: a plurality of X-ray data of a calibration object acquired with a calibration X-ray scanner over a plurality of acquisition periods, a plurality of position data and/or a plurality of movement data of an X-ray source and X-ray detector of the calibration X-ray scanner each associated with an acquisition period and determined from sensor data of a rotatable structure of the X-ray scanner that comprises the X-ray source and the X-ray detector acquired by at least one 3D sensor.

In an example, a temperature sensor and/or a humidity sensor are associated with the calibration X-ray scanner configured to acquired temperature data and/or humidity data that can be provided to the processing unit, and wherein training of the machine learning algorithm comprises utilization of the temperature data and/or humidity data.

Thus, this enables the effects of temperature and humidity, that can have an impact on vibration modes, and that can be difficult to be resolved by the 3D sensors, to be taken into account in the machine learning algorithm training. Then, when utilized for image construction of an unknown object, in addition to 3D sensor data of the source and detector, the effects of temperature and humidity can be taken into account.

According to an example, the at least one 3D sensor comprises a LIDAR sensor and/or a RADAR sensor.

Fig. 2 shows an X-ray imaging method 100 in its basic steps. The method 100 comprises:
in a positioning step 110, also referred to as step a), positioning an object between an X-ray source and an X-ray detector of a rotatable structure of an X-ray scanner;
in a moving step 120, also referred to as step b), rotationally moving the X-ray source and the X-ray detector to different intended rotational positions;
in an emitting and acquiring step 130, also referred to as step c), emitting by the X-ray source X-rays and acquiring by the X-ray detector a plurality of X-ray data of the object over a plurality of acquisition periods and providing the plurality of X-ray data of the object to the processing unit;
in an acquiring step 140, also referred to as step d), acquiring by at least one 3D sensor a plurality of sensor data of the rotatable structure, wherein each sensor data is associated with an acquisition period and providing the plurality of sensor data of the rotatable structure to the processing unit;
in a providing step 150, also referred to as step e), providing the plurality of X-ray data of the object and the plurality of sensor data of the rotatable structure to the processing unit;
in a determining step 160, also referred to as step f), determining by a processing unit a plurality of position data and/or a plurality of movement data of the X-ray source, wherein each position data and/or each movement data is associated with an acquisition period and the determining comprises utilizing the sensor data of the rotatable structure for that acquisition period;
in a determining step 170, also referred to as step g), determining by the processing unit a plurality of position data and/or a plurality of movement data of the X-ray detector, wherein each position data and/or each movement data is associated with an acquisition period and the determining comprises utilizing the sensor data of the rotatable structure for that acquisition period; and
in a determining step 180, also referred to as step h), determining by the processing unit image data of the object, the determining comprising utilizing: the plurality of X-ray data of the object, the plurality of position data and/or the plurality of movement data of the X-ray source, and the plurality of position data and/or the plurality of movement data of the X-ray detector.

In an example, the method comprises acquiring temperature data and/or humidity data with a temperature sensor and/or humidity sensor that can be provided to the processing unit, and wherein step h) comprises utilization of the temperature data and/or humidity data.

In an example, the X-ray scanner is configured to intentionally rotate the rotatable structure to move the X-ray source and the X-ray detector to different intended rotational positions about an iso-center of the X-ray scanner.

In an example, the processing unit is configured to determine the plurality of position data and/or the plurality of movement data of the X-ray source with respect to the iso-center of the X-ray scanner or another determined position.

In an example, the processing unit is configured to determine the plurality of position data and/or the plurality of movement data of the X-ray detector with respect to the iso-center of the X-ray scanner or another determined position.

In an example, the processing unit is configured to determine the plurality of position data with respect to a plurality of expected positions.

In an example, the processing unit is configured to determine the plurality of movement data with respect to a plurality of expected movements.

In an example, step h) comprises utilization of the X-ray data of the object for each acquisition period to generate a reconstructed image of the object and utilization of the position data and/or the movement data of the X-ray source for each acquisition period and the position data and/or the movement data of the X-ray detector for each acquisition period to correct the reconstructed image of the object to generate a corrected reconstructed image of the object.

In an example, step h) comprises utilization of the position data and/or the movement data of the X-ray source for each acquisition period and the position data and/or the movement data of the X-ray detector for each acquisition period to generate correction information and utilization of the X-ray data of the object for each acquisition period and the correction information to generate a corrected reconstructed image of the object.

In an example, step h) comprises simultaneous utilization of the X-ray data of the object for each acquisition period and the position data and/or the movement data of the X-ray source for each acquisition period and the position data and/or the movement data of the X-ray detector for each acquisition period to generate a corrected reconstructed image of the object.

In an example, step h) comprises utilization of the plurality of position data and/or the movement data of the X-ray source and the plurality of position data and/or the movement data of the X-ray detector to back project the plurality of X-ray data of the object during reconstruction of an image of the object.

In an example, the sensor data of the rotatable structure comprises sensor data of the X-ray detector.

In an example, the sensor data of the rotatable structure comprises sensor data of the X-ray source.

In an example, determined position data of the X-ray source comprises radial position data, and wherein determined position data of the X-ray detector comprises radial position data.

In an example, determined position data of the X-ray source is with respect to an iso-center of the X-ray scanner, and determined position data of the X-ray detector is with respect to the iso-center of the X-ray scanner.

In an example, determined movement data of the X-ray source is respect to the iso-center of the X-ray scanner, and determined movement data of the X-ray detector is with respect to the iso-center of the X-ray scanner.

In an example, determined position data of the X-ray source comprises rotational position data different to the intentional rotational positions of the X-ray source, and wherein position data of the X-ray detector comprises rotational position data different to the intentional rotational positions of the X-ray detector.

In an example, the X-ray scanner is configured to intentionally rotationally move the X-ray source to different intended radial positions with respect to the iso-center of the X-ray scanner.

In an example, the X-ray scanner is configured to intentionally rotationally move the X-ray detector to different intended radial positions with respect to the iso-center of the X-ray scanner.

In an example, determined position data of the X-ray detector comprises at least one angle between a normal perpendicular to a center of a surface of the X-ray detector and a projected line from the center of the surface of the X-ray detector that goes through the iso-center.

In an example, determined movement data of the X-ray source comprises radial movement data, and wherein determined movement data of the X-ray detector comprises radial movement data.

In an example, movement data of the X-ray source comprises rotational movement data different to the intended rotational movements of the X-ray source, and wherein movement data of the X-ray detector comprises rotational movement data different to the intended rotational movements of the X-ray detector.

In an example, method comprises storing in a memory a model configured with position data or position information and/or with movement data or movement information and/or with X-ray data and the processing unit is configured to implement the model, and wherein step h) comprises utilization of the model.

In an example the model is configured to determine geometric and/or kinematic information related to the X-ray scanner, the determination comprising utilization of the position data and/or the movement data of the X-ray source for each acquisition period and the position data and/or the movement data of the X-ray detector for each acquisition period, and wherein the geometric and/or kinematic information related to the X-ray scanner takes into account a corrected position and/or a corrected movement of the X-ray source and a corrected position and/or a corrected movement of the X-ray detector for each acquisition period.

In an example, step h) comprises implementing by the processing unit an image reconstructor to determine the image data of the object on the basis of the X-ray data of the object for each acquisition period, and the geometric and/or kinematic information related to the X-ray scanner.

In an example, step h) comprises implementing by the processing unit an image reconstructor to determine initial image data of the object on the basis of the X-ray data of the object for each acquisition period, and wherein the processing unit is configured to determine the image data of the object on the basis of the initial image data of the object, and the geometric and/or kinematic information related to the X-ray scanner.

In an example, the model is configured to determine a corrected position and/or a corrected movement of the X-ray source and a corrected position and/or a corrected movement of the X-ray detector for each acquisition period, the determination comprising utilization of the position data and/or the movement data of the X-ray source for each acquisition period and the position data and/or the movement data of the X-ray detector for each acquisition period.

In an example, step h) comprises implementing by the processing unit an image reconstructor to determine the image data of the object on the basis of the X-ray data of the object for each acquisition period, the corrected position and/or the corrected movement of the X-ray source and the corrected position and/or the corrected movement of the X-ray detector for each acquisition period.

In an example, step h) comprises implementing by the processing unit an image reconstructor and determining initial image data of the object on the basis of the X-ray data of the object for each acquisition period, and wherein determining by the processing unit the image data of the object is carried out on the basis of the initial image data of the object, the corrected position and/or the corrected movement of the X-ray source and the corrected position and/or the corrected movement of the X-ray detector for each acquisition period.

In an example, the processing unit is configured to implement a trained machine learning algorithm, and wherein step h) comprises utilization of the trained machine learning algorithm.

In an example, the machine learning algorithm was trained on the basis of: a plurality of position data and/or a plurality of movement data of the X-ray source and X-ray detector each associated with an acquisition period and determined from sensor data of the rotatable structure.

In an example, the machine learning algorithm was trained on the basis of: a plurality of position data and/or a plurality of movement data of an X-ray source and X-ray detector of the calibration X-ray scanner each associated with an acquisition period and determined from sensor data acquired by at least one 3D sensor of a rotatable structure of the X-ray scanner that comprises the X-ray source and the X-ray detector acquired by at least one 3D sensor.

In an example, the machine learning algorithm was trained on the basis of: a plurality of X-ray data of a calibration object acquired with the X-ray scanner over a plurality of acquisition periods, a plurality of position data and/or a plurality of movement data of the X-ray source and X-ray detector each associated with an acquisition period and determined from sensor data of the rotatable structure.

In an example, the machine learning algorithm was trained on the basis of: a plurality of X-ray data of a calibration object acquired with a calibration X-ray scanner over a plurality of acquisition periods, a plurality of position data and/or a plurality of movement data of an X-ray source and X-ray detector of the calibration X-ray scanner each associated with an acquisition period and determined from sensor data of a rotatable structure of the X-ray scanner that comprises the X-ray source and the X-ray detector acquired by at least one 3D sensor.

In an example, a temperature sensor and/or a humidity sensor are associated with the calibration X-ray scanner configured to acquired temperature data and/or humidity data that can be provided to the processing unit, and wherein training of the machine learning algorithm comprises utilization of the temperature data and/or humidity data.

In an example, the at least one 3D sensor comprises a LIDAR sensor and/or a RADAR sensor.

Thus, geometry/kinematic data of a rotating X-ray scanner as it acquires is derived from sensor data and is used to correct for vibrations and flexing of the scanner system. When reconstructing an image from X-ray data, geometry data in the form of positions of each detector pixel and the emission point of the X-ray source (in effect the focal spot) need to be known very accurately for each acquisition if an artefact free isotropic image with resolution is required. This geometry information is used during reconstruction to an effect back project the measured X-ray projection data, and this geometry information is presently determined from machine information, for example coming from locked data reported by motors driving the scanner from which the positions of the source and detector can be determined. However, this is inaccurate. Another present technique is to acquire in effect a calibrated geometry, where a known object is imaged in the geometries derived, and then an unknown object is imaged in exactly the same way, with same speed, accelerations, direction of movement et cetera, however this cannot account for the motions that are not reproducible, such as vibrations.

However now, the new technique described here provides sensor data from which the actual positions of the source and detector that each acquisition period can be determined, and/or geometry and kinematic information can be determined from which the reconstructed image can be corrected. This can be carried out directly, in terms of determining the positions and/or movements of the source and detector in a 3-D world coordinate positions and with all necessary degrees of freedom, or AI-based systems can be used with for example a known calibration phantom from which lidar and radar sensor based data can train the model enabling it to determine geometric and/or kinematic information when acquiring real data. In a classical approach, and can also analyses eigenmodes of the system and derive the corresponding geometry. Indeed, geometry/kinematic information or corrected position/movement data can be first derived and then utilized in an image reconstruction step to correct for the image generation. Or, image reconstruction can be carried out first, albeit then leading to an image that has artifacts, and then geometry/kinematic information or corrected position/movement data can then be utilized to correct the reconstructed image. Or indeed a new reconstruction technique can be utilized, where X-ray data and geometry/kinematic information or corrected position/movement data are utilized simultaneously to reconstruct a corrected image.

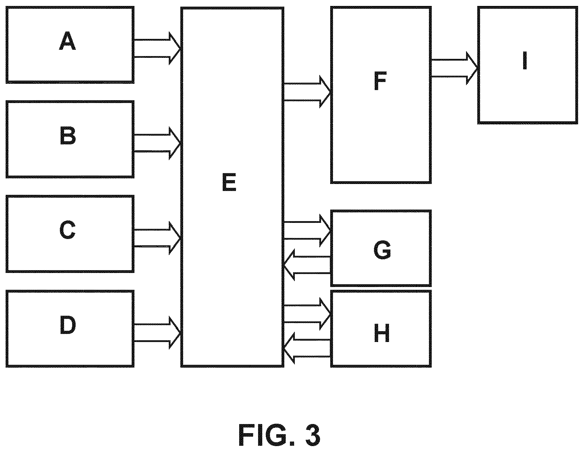

The X-ray imaging system and X-ray imaging method are explained in specific detail, where reference is made to Figs. 3-5, and where an X-ray scanner in the form of a C-arm scanner is described, but other rotational scanners could be utilized.

Fig. 3 shows a detailed high level system architecture and workflow of an exemplar X-ray imaging system. Fig.3 shows a block diagram for the data flow. In Fig. 3, A indicates one or more optical active lidar based 3-D sensors, B indicates one or more radar based 3-D sensors, C indicates position and status information of an X-ray scanner, D indicates a database of phantom calibration, E indicates an AI-based processing unit, F indicates an AI-based Reconstruction unit, G indicates the X-ray scanner, H indicates a database of service logging, and I indicates a display terminal.

Continuing with Fig. 3 the system allows the current angular speed, the 3D motion vectors and fixed parameters of the X-ray source and detector to be detected, stored and analyzed and further processed in a reconstruction algorithm. A state machine receives real/time and static input data from different input devices. 3D sensors deliver 4D point cloud data of the source and detector, which are processed by the (AI-based) state machine. Additional information are the status of the C-arm scanner (on/off, idle, X-ray tube status, voltage, water cooling...), which is used for decision control and reconstruction. Data including reference phantom calibration data may additionally be used for the (AI-based) reconstruction. The different sensors (3D video, LIDAR, RADAR) can be integrated in one unit and provide separate 3D point cloud date, which can be combined for better detection and classification of position. The same sensors may also serve additional functionality like collision avoidance and vital signs reception (e.g. respiration and heart rate). Regarding vibrational data acquired for the source and detector by the 3d sensors, differential equations can be utilized to define for example damped vibrations, and these can be used to generate a model to interpolate vibrational data to increase accuracy. This is however optional.

Thus, the X-ray imaging system has an image reconstruction unit that reconstructs an image for display purpose, from image data from a C-arm. An image distortion correction unit that provides a correction of the distortion of an image, from motion and position information of the C-arm. Here, a correction process is implemented that is derived from a calibration process of the C-arm configured with oscillation/vibration and positioning parameters of the C-arm during motions and based on 4D sensing data obtained during motions and provided by 3D sensors arranged to detect vibrations frequencies of the X-ray source and X-ray detector of the C-arm. Regarding the above, the image reconstruction unit uses the correction of the distortion to reconstruct an image.

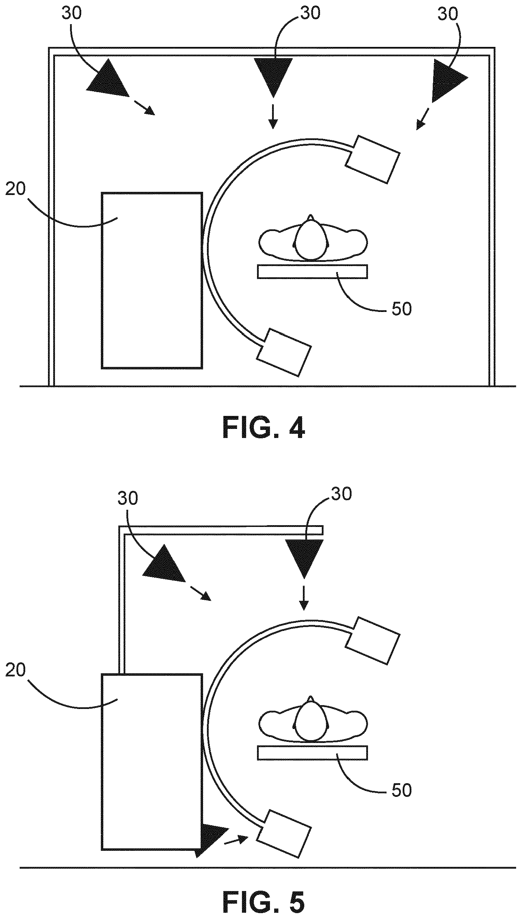

Fig. 4 shows a schematic set up of an example of an X-ray imaging system, for 3D sensing in a clinical environment. The C-arm device can rotate or a mobile device change position. Local 3 D sensing is positioned on an external frame or wall of an imaging center. Fig. 5 shows a schematic set up of an example of an X-ray imaging system, for 3D sensing in a clinical environment for mobile applications. The C-arm device can rotate or a mobile device change position. Local 3 D sensing is positioned on a holder frame, which is fixed at the diagnostic scanner. The relative position of 3 D sensing and mobile diagnostic scanner is fixed. In Figs. 4-5, 30 indicates 3D radar or Lidar sensor, 20 indicates diagnostic X-ray scanner, and 50 indicates a patient bed.

As shown in Figs. 3-5 contactless tracking and classification is provided using 3D sensing such as LIDAR or RADAR to collect 3D geometry information of the C-arm X-Ray scanner and use the information for real time calibration and improved reconstruction. Accurate monitoring of the X- Ray C - arm system geometry is enabled, and the mechanical geometry distortion, oscillation and system orientation are derived from the RADAR/LIDAR measurements. In effect the system analyses the 4D point cloud, and classifies/derives the mechanical positions for the X-ray source (tube) and detector.

Regarding the 3D sensor data of the X-ray source and detector, each pixel of 3D sensor data contains information regarding time, 3D geometrical position and dynamic parameters like velocity and acceleration. The Active 3D sensors (Radar and Lidar) are located at different positions (ceiling, wall, and bottom) and are linked with a state machine. The active sensors can be located at a frame holder fixed at the C-arm scanner itself, thus the device is mobile. Additionally, rather than have 3D sensor fixed in position, the 3D sensors can be positioned on moving parts of the X-ray scanner, and sensor data can be used to determine the position and vibration from measuring distances in relation to any fixed surrounding. However, fixed sensor measuring the position, movement and vibrations of the moving detector and source are more practicable. The computing functionality (processing unit) can be integrated in one housing (smart autonomous device) including AI based computing (a machine learning algorithm such as a trained neural network being implemented) and digital communication and wireless link to other sensors and main state machine.

To improve tracking and classification of the moving C-arm device the system is coated with a coating structured surface to absorb RADAR waves. Additionally local RADAR reflectors/markers are fixed at positions of the imaging device to provide landmarks to provide accurate sensing data for the algorithm. A Lidar based compatible structured surface coating on the C-arm system can be utilized for higher contrast and thus improved detection.

Thus, the inventors realized that they could utilize 3D sensors such as Radar and Lidar to determine the X-ray source and X-ray detector positions and movements (in effect vibrations) that are deviations from that for a perfectly rigid body (the rotating C-arm system). The motion of the source and detector parts of the C-arm are in effect tracked over and above the inherent C-arm motion (rotation) to determine vibration and motions, that are then utilized to correct for such motion induced artefacts in reconstructed image data.

To explain further, for proper 3D reconstruction of X-ray data acquired by a C-arm system, the source position (3D vector) and the position and orientation of the flat detector (3D vector + 2 tilt angles) has to be known with respect to a fixed 3D point in space (the patient). Typically, the fixed 3D point is given by the "iso-center" of the C-arm. As the C-arm system rotates the source and detector, the ideal positions of the source and detector are known. However, as the C-arm is not perfectly rigid, as it rotates and stops and accelerates and decelerates etc, it deforms and the source and detector then vibrate, and the detector can even tilt or twist. The system then monitors this vibrational motion with 3D sensors, and in a specific embodiment feeds this information into a neural network that was trained using such sensor data when acquiring X-ray data for a known object. The neural network is then able to correct for the non-ideal source and detector motion as part of a reconstruction algorithm. As part of this process, the phantom based calibration, where use is made of known 3D positions of metal beads, can also be utilized as part of the new X-ray imaging modality.

In another exemplary embodiment, a computer program or computer program element is provided that is characterized by being configured to execute the method steps of the method according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray imaging system (10), comprising:
- an X-ray scanner (20) comprising a rotatable structure, wherein the rotatable structure comprises an X-ray source and an X-ray detector, wherein the X-ray scanner is configured to rotate the rotatable structure to position the X-ray source and the X-ray detector at a plurality of different intentional rotational positions, and wherein the X-ray source is configured to emit X-rays and the X-ray detector is configured to acquire a plurality of X-ray data of an object within a plurality of acquisition periods for the plurality of different determined rotational positions, wherein each X-ray data is acquired within a different acquisition period;
- at least one 3D sensor (30); and
- a processing unit (40);
wherein, the at least one 3D sensor is configured to acquire a plurality of sensor data of the rotatable structure, wherein each sensor data is associated with a different acquisition period;
wherein, for each acquisition period of the plurality of acquisition periods the processing unit is configured to determine position data and/or movement data of the X-ray source and position data and/or movement data of the X-ray detector, wherein the determination comprises utilization of the sensor data of the rotatable structure associated with each acquisition period; and
wherein, the processing unit is configured to determine image data of the object, the determination comprising utilization of: the X-ray data of the object for each acquisition period, the position data and/or the movement data of the X-ray source for each acquisition period, and the position data and/or the movement data of the X-ray detector for each acquisition period.

2. System according to claim 1, wherein the determination of the image data of the object comprises utilization of the X-ray data of the object for each acquisition period to generate a reconstructed image of the object and utilization of the position data and/or the movement data of the X-ray source for each acquisition period and the position data and/or the movement data of the X-ray detector for each acquisition period to correct the reconstructed image of the object to generate a corrected reconstructed image of the object; or wherein the determination of the image data of the object comprises utilization of the position data and/or the movement data of the X-ray source for each acquisition period and the position data and/or the movement data of the X-ray detector for each acquisition period to generate correction information and utilization of the X-ray data of the object for each acquisition period and the correction information to generate a corrected reconstructed image of the object; or wherein the determination of the image data of the object comprises simultaneous utilization of the X-ray data of the object for each acquisition period and the position data and/or the movement data of the X-ray source for each acquisition period and the position data and/or the movement data of the X-ray detector for each acquisition period to generate a corrected reconstructed image of the object.

3. System according to any of claims 1-2, wherein the system comprises a memory storing a model configured with position data or position information and/or with movement data or movement information and/or with X-ray data and wherein the processing unit is configured to implement the model, and wherein the determination of the image data of the object comprises utilization of the model.

4. System according to claim 3, wherein the model is configured to determine geometric and/or kinematic information related to the X-ray scanner, the determination comprising utilization of the position data and/or the movement data of the X-ray source for each acquisition period and the position data and/or the movement data of the X-ray detector for each acquisition period, and wherein the geometric and/or kinematic information related to the X-ray scanner takes into account a corrected position and/or a corrected movement of the X-ray source and a corrected position and/or a corrected movement of the X-ray detector for each acquisition period.

5. System according to claim 4, wherein the processing unit is configured to implement an image reconstructor to determine the image data of the object on the basis of the X-ray data of the object for each acquisition period, and the geometric and/or kinematic information related to the X-ray scanner.

6. System according to claim 4, wherein the processing unit is configured to implement an image reconstructor to determine initial image data of the object on the basis of the X-ray data of the object for each acquisition period, and wherein the processing unit is configured to determine the image data of the object on the basis of the initial image data of the object, and the geometric and/or kinematic information related to the X-ray scanner.

7. System according to claim 3, wherein the model is configured to determine a corrected position and/or a corrected movement of the X-ray source and a corrected position and/or a corrected movement of the X-ray detector for each acquisition period, the determination comprising utilization of the position data and/or the movement data of the X-ray source for each acquisition period and the position data and/or the movement data of the X-ray detector for each acquisition period.

8. System according to claim 7, wherein the processing unit is configured to implement an image reconstructor to determine the image data of the object on the basis of the X-ray data of the object for each acquisition period, the corrected position and/or the corrected movement of the X-ray source and the corrected position and/or the corrected movement of the X-ray detector for each acquisition period.

9. System according to claim 7, wherein the processing unit is configured to implement an image reconstructor to determine initial image data of the object on the basis of the X-ray data of the object for each acquisition period, and wherein the processing unit is configured to determine the image data of the object on the basis of the initial image data of the object, the corrected position and/or the corrected movement of the X-ray source and the corrected position and/or the corrected movement of the X-ray detector for each acquisition period.

10. System according to any of claims 4-9, wherein the model is a trained machine learning algorithm.

11. System according to claim 10, wherein the machine learning algorithm was trained on the basis of: a plurality of position data and/or a plurality of movement data of the X-ray source and X-ray detector each associated with an acquisition period and determined from sensor data of the rotatable structure; or wherein the machine learning algorithm was trained on the basis of: a plurality of position data and/or a plurality of movement data of an X-ray source and X-ray detector of the calibration X-ray scanner each associated with an acquisition period and determined from sensor data acquired by at least one 3D sensor of a rotatable structure of the X-ray scanner that comprises the X-ray source and the X-ray detector acquired by at least one 3D sensor.

12. System according to claim 10, wherein the machine learning algorithm was trained on the basis of: a plurality of X-ray data of a calibration object acquired with the X-ray scanner over a plurality of acquisition periods, a plurality of position data and/or a plurality of movement data of the X-ray source and X-ray detector each associated with an acquisition period and determined from sensor data of the rotatable structure; or wherein the machine learning algorithm was trained on the basis of: a plurality of X-ray data of a calibration object acquired with a calibration X-ray scanner over a plurality of acquisition periods, a plurality of position data and/or a plurality of movement data of an X-ray source and X-ray detector of the calibration X-ray scanner each associated with an acquisition period and determined from sensor data of a rotatable structure of the X-ray scanner that comprises the X-ray source and the X-ray detector acquired by at least one 3D sensor .

13. System according to any of claims 1-12, wherein the at least one 3D sensor comprises a LIDAR sensor and/or a RADAR sensor.

14. An X-ray imaging method (100), comprising:
a) positioning (110) an object between an X-ray source and an X-ray detector of a rotatable structure of an X-ray scanner;
b) rotationally moving (120) the X-ray source and the X-ray detector to different intended rotational positions;
c) emitting by the X-ray source X-rays and acquiring (130) by the X-ray detector a plurality of X-ray data of the object over a plurality of acquisition periods and providing the plurality of X-ray data of the object to the processing unit;
d) acquiring (140) by at least one 3D sensor a plurality of sensor data of the rotatable structure, wherein each sensor data is associated with an acquisition period and providing the plurality of sensor data of the rotatable structure to the processing unit;
e) providing (150) the plurality of X-ray data of the object and the plurality of sensor data of the rotatable structure to the processing unit;
f) determining (160) by a processing unit a plurality of position data and/or a plurality of movement data of the X-ray source, wherein each position data and/or each movement data is associated with an acquisition period and the determining comprises utilizing the sensor data of the rotatable structure for that acquisition period;
g) determining (170) by the processing unit a plurality of position data and/or a plurality of movement data of the X-ray detector, wherein each position data and/or each movement data is associated with an acquisition period and the determining comprises utilizing the sensor data of the rotatable structure for that acquisition period; and
h) determining (180) by the processing unit image data of the object, the determining comprising utilizing: the plurality of X-ray data of the object, the plurality of position data and/or the plurality of movement data of the X-ray source, and the plurality of position data and/or the plurality of movement data of the X-ray detector.

15. A computer program element for controlling a system according to one of claims 1 to 13, which when executed by a processor is configured to carry out the method of claim 14.
